# EUROPEAN PATENT APPLICATION

(11) **EP 3 757 203 A1**
(43) Date of publication of application: **30.12.2020**
(21) Application number: 19757055.9
(22) Date of filing: 21.02.2019
(51) Int. Cl.: C12M 1/00, C12M 1/26, C12M 3/00

(54) **CELL CULTURE CONTAINER, CELL CULTURE CONTAINER MANUFACTURING METHOD, CELL COLLECTION SYSTEM, AND CELL ACQUISITION METHOD**

(30) Priority: 21.02.2018 JP 2018028921
(71) Applicant: UNIVERSITY PUBLIC CORPORATION OSAKA, Osaka-shi Osaka 5998531 (JP); Tokyo Women's Medical University, Tokyo 162-8666 (JP); NIKON CORPORATION, Minato-ku Tokyo 108-6290 (JP)
(72) Inventor: KOJIMA Chie, Sakai-shi, Osaka 599-8531 (JP); SHIMIZU Tatsuya, Tokyo 162-8666 (JP); HARAGUCHI Yuji, Tokyo 162-8666 (JP); KAWANO Takeshi, Tokyo 108-6290 (JP); TAKATSUKA Kenji, Tokyo 108-6290 (JP); YOKOYAMA Kaede, Tokyo 108-6290 (JP); TAKI Yusuke, Tokyo 108-6290 (JP)
(74) Representative: Hoffmann Eitle
(86) International application number: PCT/JP2019/006495
(87) International publication number: WO 2019/163877

(57) **Abstract**

A cell culture container is provided which is filled with a cell culture substrate (14) including a gel layer formed of a gel (15) capable of being denatured by heating and a gold nanoparticle layer (13) formed on one surface of the gel layer, in which a cell is cultured on a side of the one surface or a side of the other surface in the cell culture substrate. A method for manufacturing a cell culture container, including: a step of filling the cell culture container with a gel capable of being denatured by heating; and a step of forming a gold nanoparticle layer on one surface of the gel. A method for acquiring a cell, including: a step of selecting a cell to be acquired from cells placed in the cell culture container; a step of irradiating a cell culture substrate in the vicinity of the selected cell with light; and a step of recovering the selected cell.

## Description

### TECHNICAL FIELD

The present invention relates to a cell culture container, a method for manufacturing a cell culture container, a cell recovery system, and a method for acquiring cells.

Priority is claimed on Japanese Patent Application No. 2018-28921, filed February 21, 2018, the content of which is incorporated herein by reference.

### BACKGROUND ART

In order to recover cells cultured using a culture substrate more efficiently, for example, there has been reported a method of culturing cells above a culture substrate including a thermosensitive polymer and recovering the cells using a change in temperature (refer to Patent Document 1).

### Citation List

### Patent Literature

[Patent Document 1]
Japanese Unexamined Patent Application, First Publication No. HI 1-349643

### DISCLOSURE OF INVENTION

According to a first aspect of the present invention, a cell culture container is filled with a cell culture substrate including a gel layer formed of a gel capable of being denatured by heating and a gold nanoparticle layer formed on one surface of the gel layer. A cell is cultured on a side of the one surface or a side of the other surface in the cell culture substrate.

According to a second aspect of the present invention, a method for manufacturing a cell culture container, includes: a step of filling a cell culture container with a gel capable of being denatured by heating; and a step of forming a gold nanoparticle layer on one surface of the gel.

According to a third aspect of the present invention, a cell recovery system includes: the cell culture container according to the first aspect; and a suction pump connected to the cell culture container.

According to a fourth aspect of the present invention, a method of acquiring cells, includes: a step of selecting cells to be acquired from cells placed in the cell culture container according to the first aspect; a step of irradiating a cell culture substrate in the vicinity of the selected cells with light; and a step of recovering the selected cells.

### BRIEF DESCRIPTION OF DRAWINGS

Fig. 1 is a cross-sectional view of a cell culture container in a first embodiment.
Fig. 2 is a cross-sectional view of a cell culture container in a second embodiment.
Fig. 3 is a cross-sectional view of a cell culture container in a third embodiment.
Fig. 4 is a cross-sectional view of a cell culture container in a fourth embodiment.
Fig. 5 is a conceptual diagram illustrating the constitution of a cell acquisition system including the cell culture container in the embodiment.
Fig. 6 is a diagram schematically illustrating a method for acquiring cells using the cell culture container in the fourth embodiment.
Fig. 7 is a conceptual diagram illustrating the constitution of the cell culture container in the embodiment.
Fig. 8 is a conceptual diagram illustrating the constitution of the cell recovery system in the embodiment.
Fig. 9 is a flowchart describing a flow of a method for acquiring cells using the cell culture container in the embodiment.

### BEST MODE FOR CARRYING OUT THE INVENTION

### (First Embodiment)

A cell culture container, a method for manufacturing a cell culture container, a cell recovery system, a method for acquiring cells, and the like in an embodiment will be described below with reference to the drawings as appropriate.

It is necessary to efficiently detach and recover specific cells such as cells that have been succeeded in gene transfer among cells cultured above a cell culture substrate in many cases.

The present inventors found that by culturing cells in a cell culture container filled with a cell culture substrate containing a gel layer formed of a gel capable of being denatured by heating and a gold nanoparticle layer formed on one surface of the gel layer, and thereafter irradiating the cell culture container with light from the outside for an extremely short period of time, the gel in the cell culture substrate is denatured very efficiently. Accordingly, it was found that the cells are detached from the cell culture substrate or the cells are detached from the culture container together with the cell culture substrate, whereby the cells can be efficiently recovered by suction or the like without causing any damage.

Particularly, it was found that in a case where the cell culture container has a plurality of wells, the cell culture substrate is contracted by the light from the outside and detached from the well, and the cell culture substrate is taken out by suction or the like, whereby without damaging target cells adhering to the cell culture substrate, the cells can be efficiently recovered.

Furthermore, it was found that by manufacturing the cell culture substrate by a method for filling the cell culture container with the gel and forming the gold nanoparticle layer on one surface of the gel, the cell culture container can be manufactured without wasting gold nanoparticles.

By using the cell culture container of the present embodiment, specific and efficient recovery of target cells is realized.

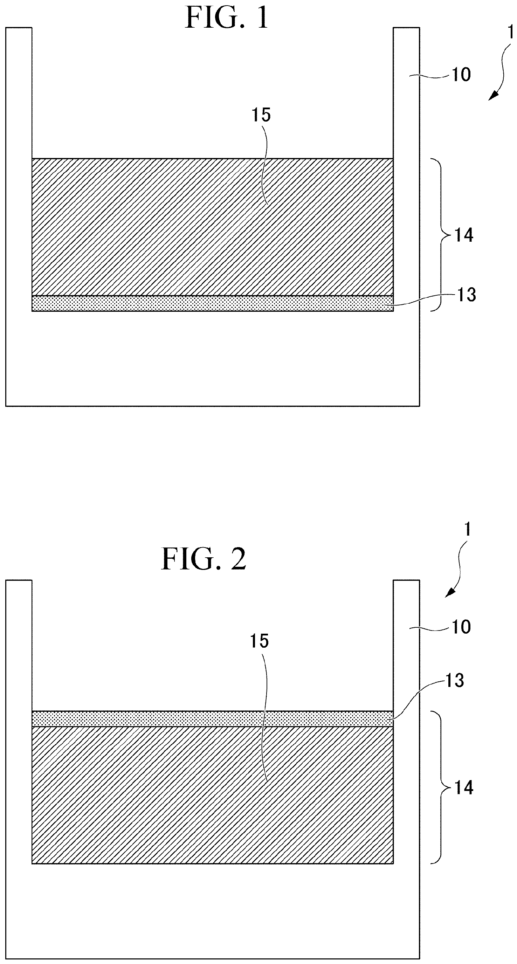

Fig. 1 is a cross-sectional view of a first embodiment of the cell culture container of the present embodiment. A cell culture container 1 is filled with a gel 15 capable of being denatured by heating, and a gold nanoparticle layer 13 is formed on the lower surface side of the gel 15. In the first embodiment, the cell culture container 1 has one opening. The cell culture container 1 may have or may not have a lid such as that on a Petri dish.

Gold nanoparticles forming the gold nanoparticle layer 13 can be particles having excellent photothermal conversion characteristics, and particularly, may have sizes in which surface plasmon resonance absorption (SPR) occurs. A volume-average diameter of the gold nanoparticles measured using a laser diffraction type particle size distribution analyzer can be set to, for example, 1 nm or more and less than 200 nm, 10 nm or more and less than 100 nm, and 30 nm or more and less than 70 nm.

A solution containing gold nanoparticles having a predetermined volume-average diameter can be obtained, for example, as in the method described in Japanese Unexamined Patent Application, First Publication No. 2013-233101, by growing seed nuclei made of gold in the presence of a reducing agent (ascorbic acid, hydroquinone, citric acid, or the like) in a solution containing gold ions (HAuCl₄ or the like).

The volume-average diameter of the gold nanoparticles can be measured by the laser diffraction type particle size distribution analyzer.

In the case of a simple measurement without using the particle size distribution analyzer, an image may be taken by using a transmission electron microscope, and measurement and calculation may be performed using analysis software or the like.

The gel 15 denatures when a temperature thereof rises from room temperature to a predetermined temperature. In the present embodiment, the term "denaturation" means that the gel 15 undergoes a structural change that causes easy detachment of cells from the gel 15 or easy detachment of the gel 15 from the cell culture container 1 due to a rise in temperature. In a case where the gel 15 is a collagen gel or a gelatin gel, the term "denaturation" includes, for example, solation, aggregation, and decomposition into small molecules, and the like which are states caused due to changes in the secondary or tertiary structures of collagen proteins. The temperature at which the gel 15 denatures is, for example, 60°C or lower, 50°C or lower, or 40°C or lower. Although a material of the gel 15 is not particularly limited as long as it enables cells to be obtained through detachment from the gel 15, which will be described later, for example, the material of the gel 15 may be a collagen gel or gelatin gel. It is also possible to use a gel to which a crosslinking agent is added or a gel obtained by mixing two or more kinds of polymers. Alternatively, it is possible to use a gel in which gold nanoparticles are dispersed as the gel 15.

Although a method for forming the gold nanoparticle layer 13 on the lower surface side of the gel 15 is not particularly limited, for example, first, a solution containing gold nanoparticles is poured into the cell culture container 1 and dried overnight or the like to cause a liquid component in the solution to evaporate, thereby forming the gold nanoparticle layer 13 on the bottom surface of the cell culture container 1. Subsequently, the gel 15 is formed on the gold nanoparticle layer 13. For example, in the case of a collagen gel, the gel 15 can be formed by supplying a collagen solution into the cell culture container 1 and allowing the collagen solution to be gelated.

At this time, the concentration of the gold nanoparticle solution poured into the cell culture container 1 may be set to, for example, 100 µM or more and less than 2000 µM, 250 µM or more and 1500 µM or less, or 250 µM or more and 800 µM or less. When the concentration of the gold nanoparticle solution is low, the calorific value of the gold nanoparticles is low, and the contraction rate of the gel 15 decreases, so that the probability of successful detachment from the gel 15 tends to decrease. On the other hand, when the concentration of the gold nanoparticle solution is high, local rise in temperature increases. Thus, it difficult to control the temperature and cytotoxicity tends to increase. The gold nanoparticles may be further stabilized using a protective agent, for example, by being encapsulated in a dendrimer or modified with a molecules having an affinity for a gel.

### (Second Embodiment)

Fig. 2 is a cross-sectional view of a second embodiment of the cell culture container. The cell culture container 1 is filled with the gel 15 capable of being denatured by heating, and the gold nanoparticle layer 13 is formed on the upper surface side of the gel 15. Features other than the placement of the gel 15 and the gold nanoparticle layer 13 are the same as those of the first embodiment illustrated in Fig. 1.

Although a method for preparing the cell culture substrate 14 is not particularly limited, for example, first, the cell culture container 1 is filled with the gel 15. A method for filling the gel 15 can be performed in the same manner as in the first embodiment. A solution containing gold nanoparticles is poured thereon, and dried overnight or the like to cause a liquid component in the solution to evaporate, thereby forming the gold nanoparticle layer 13 and preparing the cell culture substrate 14.

Cells can be placed on the upper surface of the cell culture substrate 14 in which the gold nanoparticle layer is formed on the upper surface or the lower surface of the gel 15, for example, by pouring a liquid containing the cells into the cell culture container 1. The number of cells to be poured into the cell culture container 1 can be controlled by adjusting the concentration of the liquid containing the cells.

In addition, the cell culture container 1 may be one having a light-transmitting characteristics. Accordingly, the cell culture substrate 14 can be irradiated with light even through the bottom of the cell culture container 1. The cell culture container 1 can be configured in any shape as long as the characteristics of the cell culture container 1 of the present embodiment are not impaired.

### (Third Embodiment)

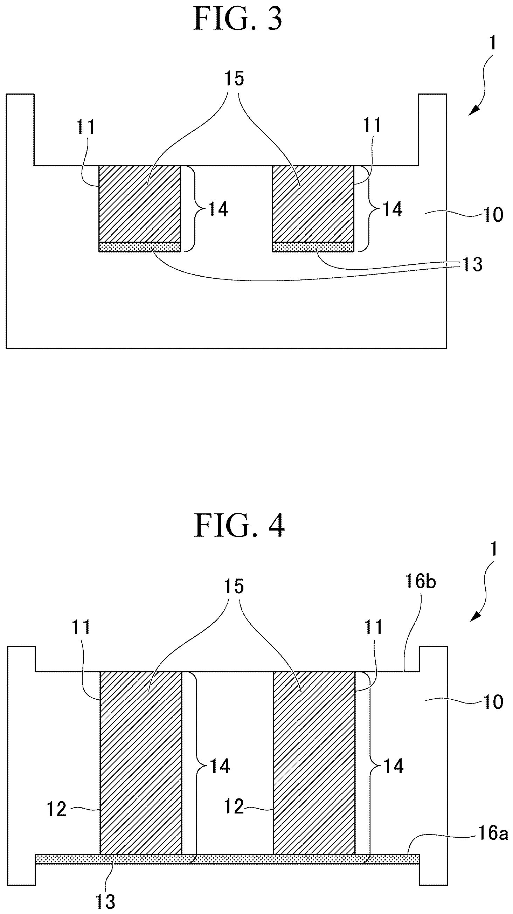

Fig. 3 is a cross-sectional view of a third embodiment of the cell culture container 1 of the present embodiment. In the present embodiment, the cell culture container 1 has the plurality of wells 11. In the third embodiment, each of the wells is filled with the gel 15 above the gold nanoparticle layer 13.

The formation of the gold nanoparticle layer 13 and the filling of the gel 15 can be performed in the same manner as in the first embodiment.

Cells can be placed above the cell culture substrate 14 in which the gel 15 is laminated on the gold nanoparticle layer 13, for example, by pouring a liquid containing cells into the cell culture container 1. As another method, an ink-jet printer may be used to seed cells in each of the wells 11.

The cell culture container 1 may have a configuration in which about one cell is placed per well. Accordingly, cells can be selected and recovered one by one, and thus only desired cells can be recovered. The term "about one cell is placed per well" means that, when the liquid containing the cells is poured into the cell culture container 1, among the wells 11 in which the cells are placed, the number of wells 11 in which only one cell is placed is the largest, and the wells 11 in which two or more cells are placed or the wells in which no cell is placed may also be present.

An example of a method for a configuration in which about one cell is placed per well is to adjust the diameter of the wells 11. Examples of the diameter at which about one cell is placed per well include a diameter of one or more times and less than three times or 1.3 or more times and less than two times the average diameter or the average longest diameter of the cells to be cultured. The diameter of each well of the cell culture container 1 may be, for example, 10 µm to 500 µm, 20 µm to 400 µm, or 30 µm to 300 µm. When the diameter of the well is too large, denaturation due to light irradiation tends not to occur easily.

Another method for the configuration in which about one cell is placed per well is to adjust the concentration of the liquid containing the cells.

As still another method for the configuration in which about one cell is placed per well is to adjust the distance between adjacent wells 11 (the minimum distance between the openings of adjacent wells 11).

The cell culture container 1 may have any number of wells 11. For example, the wells 11 can be arranged in an array (microarray) in the cell culture container 1. At this time, it is possible to adjust the number of cells placed per well by adjusting the distance between adjacent wells 11.

As described above, about one cell may be seeded in each well using an ink-jet printer.

The configuration in which about one cell is placed per well can be appropriately designed by those skilled in the art by at least one of the above-mentioned methods and/or other methods.

In addition, the cell culture container 1 may be one having a light-transmitting characteristics. Accordingly, the cell culture substrate 14 can be irradiated with light also through the bottom of the well 11. The cell culture container 1 can be constituted in any shape as long as the characteristics of the cell culture container 1 of the present embodiment are not impaired.

Although the gel 15 is filled above the gold nanoparticle layer 13 in the third embodiment, the gold nanoparticle layer 13 may be formed above the gel 15 as in the second embodiment also in the case where the cell culture container 1 has a microarray shape.

### (Fourth Embodiment)

Fig. 4 is a cross-sectional view of a fourth embodiment of the cell culture container 1 of the present embodiment. In the present embodiment, the cell culture container 1 has a plurality of wells 11, and the well 11 of the cell culture container 1 has a through-hole 12 at the bottom. The diameter of the through-hole 12 may be the same as or smaller than the diameter of the well 11. In Fig. 4, the diameters of the through-hole 12 and the well 11 are the same. Furthermore, in Fig. 4, the well 11 and the through-hole 12 are fully filled with the cell culture substrate 14, but the cell culture substrate 14 may not fully fill the well 11 and the through-hole 12. By irradiating the cell culture substrate 14 filling the well 11 with light, the heat generated by the gold nanoparticles present at the lower surface of the cell culture substrate 14 causes the gel 15 to contract, so that the cell culture substrate 14 is detached from the wall surface of the well 11. The detached cell culture substrate 14 can be recovered from the through-hole 12 by suction or the like.

Although a method for manufacturing the cell culture container 1 according to the fourth embodiment is not particularly limited, for example, first, each well 11 and the through-hole 12 are filled with the gel 15, the cell culture container 1 is placed in a state turned upside down from Fig. 4 (so that a surface 16a faces upward), and a solution containing gold nanoparticles is placed above the gel. A liquid component in the solution is caused to evaporate by drying overnight or the like, thereby forming the gold nanoparticle layer 13. Thereafter, the cell culture container 1 may be turned upside down so that a surface 16b thereof faces upward. Although a method for filling each well 11 with the gel 15 is not particularly limited, for an example, a method for forming a layer of the gel 15 in another container, and pressing the surface 16b of the cell culture container 1 against the layer to insert the gel 15 into the well 11 is mentioned.

In the fourth embodiment, although the gold nanoparticle layer 13 is formed on the lower side (the surface 16a side) of the gel 15, the gold nanoparticle layer 13 may be formed on the upper surface (on the same side as the cells are cultured, that is, the surface 16b side) of the gel 15.

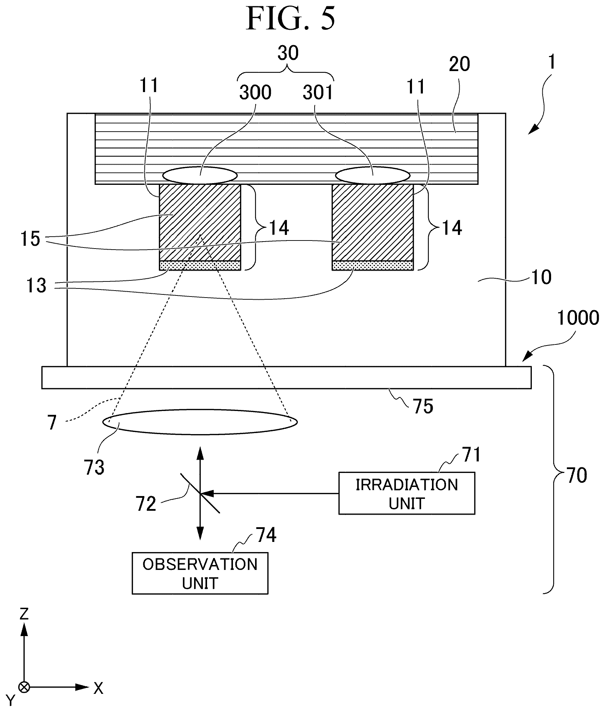

Fig. 5 is a conceptual diagram illustrating a cell recovery system 1000 which acquires cells using the cell culture container of the third embodiment. The cell acquisition system 1000 includes the cell culture container 1 and an inverted microscope 70.

Cells 30 cultured in another culture container are poured into the cell culture container 1 together with a culture solution 20. The culture solution 20 may be replaced or diluted with a culture solution, a buffer solution, or the like. The cells 30 poured into the cell culture container 1 may have a concentration such that one cell is placed per well when the cells 30 are poured into the cell culture container 1.

The cells 30 placed on the upper surface of the cell culture substrate 14 filling each well 11 of the cell culture container 1 are cultured on the upper surface of the cell culture substrate 14 of each well 11. Among the cells 30 cultured on the upper surface of the cell culture substrate 14 filling each well 11, the cells to be isolated and acquired are referred to as selected cells 300, and the other cells are referred to as non-selected cells 301. Although the form of the selected cell 300 is not particularly limited, for examples a cell in which a gene or the like has been appropriately modified and characteristics have been revealed using discriminating means such as a reporter gene, a cell in which initialization or appropriate differentiation, and the like are induced and which has characteristics capable of being discriminated structurally, and the like may be used.

The selected cell 300 may be a single cell 30 as described above, a plurality of cells 30, or a colony constituted to include a plurality of cells 30.

The inverted microscope 70 includes an irradiation unit 71, a dichroic mirror 72, a lens system 73, an observation unit 74, and a support base 75.

The cell recovery system 1000 may be constituted using an upright microscope.

The irradiation unit 71 emits a laser beam. A wavelength of the laser beam emitted from the irradiation unit 71 is set to a wavelength range in which the gold nanoparticles in the gold nanoparticle layer 13 exhibit a photothermal conversion characteristics. Particularly, the wavelength may be set in a wavelength range in which surface plasmon resonance absorption (SPR) occurs. The laser beam irradiated to the cell culture substrate 14 may have a wavelength and energy in which significant damage is not caused in the cells on the upper surface of the cell culture substrate 14 when irradiated to the cell culture substrate 14. The wavelength of the laser beam emitted from the irradiation unit 71 is set to, for example, 400 nm or more and less than 1200 nm, 450 nm or more and less than 900 nm, 532 nm, or the like. An output of the laser beam incident on the cell culture container 1 can be set to, for example, 0.1 mW or more and less than 1000 mW, and 0.4 mW or more and less than 100 mW. The wavelength and energy of the laser beam are appropriately adjusted so that a temperature of the gel contained in the cell culture substrate 14 efficiently increases and the gel efficiently denatures without damaging the cells. The light emitted from the irradiation unit 71 is incident on the dichroic mirror 72.

The light emitted from the irradiation unit 71 is not particularly limited to laser beam as long as the cell culture substrate 14 in the vicinity of the selected cells 300 can be selectively irradiated, and may be non-coherent monochromatic light or light in a certain wavelength range.

The dichroic mirror 72 reflects the laser beam from the irradiation unit 71, visible light from the cell culture container 1 is transmitted through the dichroic mirror 72 and emitted to the observation unit 74. A light direction of the laser beam reflected by the dichroic mirror 72 is adjusted so that an appropriate position is irradiated with the laser beam by a galvanometer mirror (not illustrated) or the like and the laser beam is transmitted through the lens system 73 and incident on the cell culture container 1. The laser beam incident on the cell culture container 1 is transmitted through the bottom of the cell culture container 1 and then converges at a predetermined position on the cell culture substrate 14. In Fig. 5, the converged laser beam 7 is schematically illustrated using a dot-dashed line.

The constitution of the irradiation optical system of the laser beam 7 is not particularly limited as long as the laser beam 7 can converge to a predetermined position.

The convergence position of the laser beam 7 on the cell culture substrate 14 can be, for example, any position on the cell culture substrate 14 below the selected cells 300 without damaging the selected cells 300, and can be determined, for example, based on the spot diameter of the laser beam 7, point spread function (PSF), and parameters based on PSF. In order to reduce the influence on the selected cells 300, the convergence position may be the gold nanoparticle layer 13 itself or the vicinity of the gold nanoparticle layer 13. Accordingly, an excellent photothermal effect is obtained, and cytotoxicity due to the heat generated by the gold nanoparticles is reduced, which is also suitable for the case where the selected cells 300 are cells sensitive to heat.

The convergence position of the laser beam 7 on the cell culture substrate 14 may be a position within the gel 15 where the distance to the selected cell 300 is 100 µm or more. Furthermore, the convergence position of the laser beam on the cell culture substrate 14 may be a position close to the wall surface of the well 11 instead of the central portion of the cell culture substrate 14.

When the gold nanoparticles in the gold nanoparticle layer 13 photothermally convert the laser beam 7 from the irradiation unit 71 and the gel in the vicinity of the convergence position of the laser beam 7 denatures, the gel to which the selected cells 300 adhere contracts, and detaches from the wall surface of the well 11. Therefore, by pouring a fluid such as a culture solution or a buffer solution into the cell culture container 1, the cell culture substrate 14 having the selected cells 300 adhering thereto can be suspended in the fluid and recovered together with the fluid.

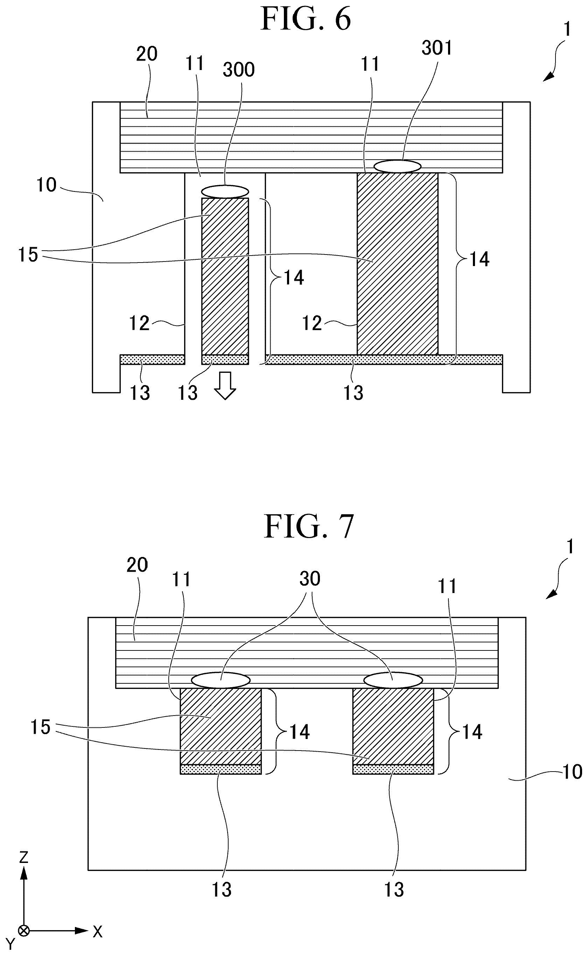

The contracted cell culture substrate 14 can also be taken out from the through-hole 12 below the well 11 by suction using a suction pump or the like (see Fig. 6).

The observation unit 74 includes an eyepiece or the like, and enables a user to observe visible light from the cell culture container 1 lit by a lighting (not illustrated). The user can see the visible light from the cell culture container 1 and appropriately position the cell culture container 1 by moving the support base 75 or the like.

A constitution in which an image of the cell culture container 1 is acquired by performing laser scanning using a laser beam source and a galvanometer mirror different from those of the irradiation optical system of the laser beam 7 and displayed on a display device (not illustrated) may also be used.

The support base 75 supports the cell culture container 1. The support base 75 can move in each of XYZ directions using a moving mechanism (not illustrated), and thus the cell culture container 1 can be adjusted to an arbitrary position. The support base 75 is constituted to include, for example, glass having a transparent heating element formed thereon, the laser beam 7 is transmitted to the bottom of the cell culture container 1, and a temperature of the entire cell culture container 1 is controlled.

Alternatively, in accordance with a size, a structure, and the like of the cell culture container 1, a support base 75 having an opening is formed in a portion serving as an optical path of the laser beam 7 may also be used.

Fig. 7 illustrates a cross-sectional view of an XZ plane of the cell culture container 1 in the third embodiment.

In the cell culture substrate 14 of the present embodiment, the thickness of the gel 15 can be set to, for example, 0.05 mm or more, 0.05 mm or more and 1.7 mm or less, or 0.1 mm or more and 1.2 mm or less. If the thickness of the gel 15 is too thin, it tends to be difficult to make the gel 15 uniform or flat. On the other hand, if the thickness of the gel 15 is too thick, it may take a long period of time to irradiate the gel 15 with light to contract the gel 15.

Here, the thickness of the gel 15 is the thickness of the cell culture substrate 14 in a depth direction of the well 11, for example, the thickness along the central axis of the well 11.

### (Cell Recovery System)

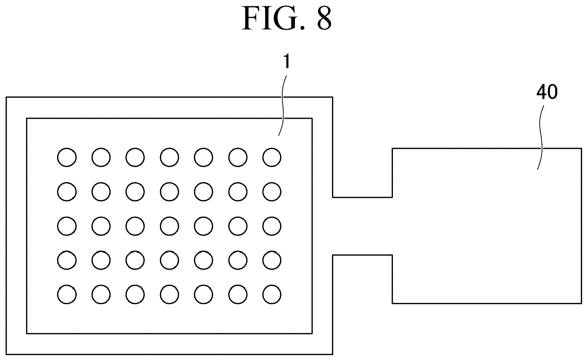

Fig. 8 illustrates a schematic diagram of the cell recovery system of the present embodiment. Although Fig. 8 illustrates the cell recovery system using the cell culture container 1 in the third and fourth embodiments, the cell culture container 1 may also be the cell culture container in the first and second embodiments.

The cell recovery system of the present embodiment includes the cell culture container 1 of the present embodiment and a suction pump 40 connected to the cell culture container 1. By connecting the suction pump 40 to the cell culture container 1, it is possible to recover by suction the cell culture substrate 14 which has been contracted by being irradiated with the laser beam 7 and has the selected cells 300 adhering thereto.

The cell culture container 1 and the suction pump 40 may be connected by any connection method as long as the selected cells 300 can be suctioned or the cell culture substrate 14 which has the selected cells 300 adhering thereto and has been contracted by light can be suctioned. For example, connection methods are mentioned in which the suction pump 40 is connected to the upper surface of the cell culture container 1 according to the third embodiment and suctions the selected cells 300 together with the culture solution 20, and in which the suction pump 40 is connected to the through-hole 12 of the cell culture container 1 according to the fourth embodiment and suctions from the through-hole 12 the cell culture substrate 14 which has the selected cells 300 adhering thereto and has been contracted by light, as illustrated in Fig. 6.

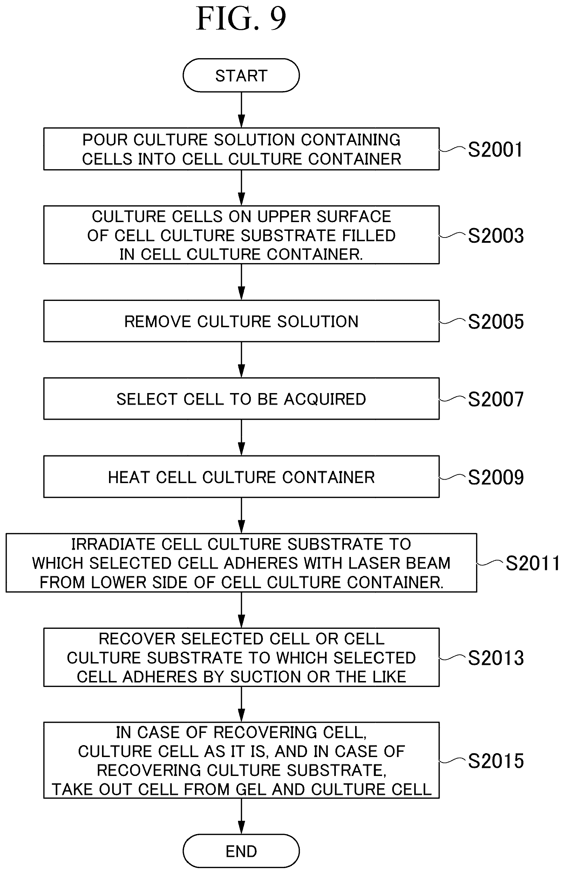

Fig. 9 is a flowchart describing a flow of the method for acquiring and cells and a method for producing cells using the cell culture container 1 of the present embodiment. In Step S2001, a culture solution containing cells is poured into the cell culture container 1. In a case where the concentration of the cells in the culture solution is high, the culture solution containing the cells may be diluted with a culture solution or the like before the culture solution containing the cells is poured into the cell culture container 1. In a case where the cell culture container 1 has a plurality of wells, the culture solution may be diluted so that one cell is placed per well of the cell culture container. When Step S2001 has ended, the process proceeds to Step S2003.

In Step S2003, the cells are cultured above the upper surface of the cell culture substrate 14 filled in the cell culture container. When Step S2003 has ended, the process proceeds to Step S2005. In Step S2005, the culture solution is removed by suction. After removing the culture solution, the upper surface of the cell culture substrate 14 filled in the cell culture container 1 may be washed. Washing is performed using PBS or the like, and unnecessary suspended substances, precipitates, and the like are removed. When Step S2005 has ended, the process proceeds to Step S2007.

In Step S2007, a cell 30 (selected cell 300) to be acquired is selected from among the cells 30 cultured on the upper surface of the cell culture substrate 14. If necessary, a cell 30 in which a fluorescent protein is expressed or a cell 30 having structural characteristics is selected. When Step S2007 has ended, the process proceeds to Step S2009. In Step S2009, a temperature of the support base 75 is appropriately adjusted, and the cell culture container 1 is heated to a temperature lower than a temperature at which the total denaturation of the cell culture substrate 14 occurs, for example, 37°C or higher and lower than 40°C. The heating of the cell culture container 1 may be performed by placing the cell culture container 1 in an incubator whose temperature can be controlled. By rising the temperature of the cell culture container 1 before the irradiation with the laser beam 7, the irradiation time of the laser beam 7 can be shortened and the cytotoxicity to the selected cell 300 can be reduced.

The order of Step S2009 and Step S2007 may be reversed. In any case, after Step 2009 has ended, the process may proceed to the subsequent step (Step S2007 or Step S2011), or the step after the subsequent step may be performed while continuing Step S2009.

In Step S2011, the convergence position in the cell culture substrate 14 to which the selected cell 300 adheres is irradiated with the laser beam 7 from the lower side of the cell culture container 1. By irradiating the laser beam 7 from the lower side of the cell culture container 1, it is possible to prevent direct light from coming into contact with the selected cell 300 and causing cell damage. By irradiating the laser beam, the heat generated by the gold nanoparticles in the gold nanoparticle layer 13 causes the gel 15 in the vicinity the selected cell 300 to be denatured and contract, and weakens the binding between the selected cell 300 and the gel, so that the selected cell 300 is detached from the cell culture substrate 14 or the cell culture substrate 14 is detached from the wall surface of the well 11 together with the cell. When the selected cell 300 or the cell culture substrate 14 is detached from the wall surface of the well 11, the process proceeds to Step S2013.

In a case of selecting a plurality of cells, a plurality of cells may be selected in Step S2007, the cell culture container 1 may be heated in Step S2009, and the plurality of cells selected in Step S2011 may be irradiated with laser beam. Alternatively, first, Step S2009 may be performed to heat the cell culture container 1, and while suspending the heating or continuing the heating, selection of the cells in Step S2007 and irradiation with the laser beam in Step S2011 may be repeatedly performed until all of the selected cells may be irradiated with a laser.

In Step S2013, in a case where the fluid such as the culture solution or the buffer solution is sufficiently present in the cell culture container 1, the fluid is left as it is, and in a case where the amount of the fluid is insufficient, the fluid such as the culture solution or the buffer solution is poured into the cell culture container 1 so that the selected cell 300 or the cell culture substrate 14 detached from the wall surface of the well 11 of the cell culture container 1 is suspended in the fluid. Therefore, the selected cell 300 can be recovered by recovering the fluid by inclining the cell culture container 1 or suctioning the fluid or the like. In a case where the selected cell 300 adheres to the cell culture substrate 14, the selected cell 300 can be recovered together with the cell culture substrate 14.

Alternatively, in a case where the cell culture container 1 has the plurality of wells 11 and the well 11 has the through-hole 12 at the bottom, the contracted cell culture substrate 14 may be recovered from the through-hole 12 below the well 11 by suction using a suction pump or the like. When Step S2013 has ended, the process proceeds to Step S2015.

In Step S2015, in a case where the selected cell 300 is recovered in Step S2013, the recovered cell 300 may be placed as it is in another culture medium or the like to be cultured, and in a case where the cell culture substrate 14 having the selected cell 300 adhering thereto may be recovered in Step S2013, the gel of the recovered cell culture substrate 14 having the selected cell 300 adhering thereto may be dissolved using collagenase or the like, the selected cell 300 having adhered to the cell culture substrate 14 may be recovered from the cell culture substrate 14, and the recovered selected cell 300 may be placed in another culture medium or the like to be cultured. When Step S2015 has ended, the process ends. In addition, when the selected cell 300 is recovered in Step S2013, the process may return to Step S2007 to acquire another cell 30 as the selected cell 300. The recovered selected cells may be directly used for various purposes such as clinical use, research, and industrial use.

According to the above-described embodiments, the following effects can be obtained.
(1) The cell culture container 1 of the present embodiment is filled with the cell culture substrate 14 including the gel layer formed of the gel 15 capable of being denatured by heating and the gold nanoparticle layer 13 formed on one surface of the gel layer. By culturing the cell on a side of the one surface or a side of the other surface in the cell culture container 1 and irradiating the cell culture substrate 14 with light, the gold nanoparticles can be caused to generate heat with higher efficiency compared to a case where the gold nanoparticles are dispersed in the gel 15, so that it is possible to detach the selected cell 300 efficiently from the cell culture substrate 14 and recover the selected cell 300. In addition, particularly, in a case where the gold nanoparticle layer is formed on the lower surface of the gel layer, the place where the gold nanoparticles generate heat by light irradiation is far from the cell, so that it is possible to reduce cell damage due to heat.
(2) In the cell culture container 1 in the embodiment, the cell culture container 1 has the plurality of wells 11. Each of the wells of the cell culture container 1 is filled with the cell culture substrate 14 including the gel layer formed of the gel 15 capable of being denatured by heating and the gold nanoparticle layer 13 formed on one surface of the gel layer. When the cell is cultured on the upper surface of the cell culture container 1, the cell culture substrate 14 having the selected cell 300 adhering thereto is contracted by irradiating the cell culture substrate 14 with light for an extremely short period of time compared to the case where the gold nanoparticles are dispersed in the gel 15, so that it is possible to detach the cell culture substrate 14 from the well 11. By recovering the cell culture substrate 14 which has been contracted by light and has the selected cell 300 adhering thereto by suction or the like, it is possible to recover the selected cell 300 together with the cell culture substrate 14. Therefore, it is possible to increase the number of cells recovered per unit time without damaging the selected cells 300. In addition, particularly, in a case where the gold nanoparticle layer is formed on the lower surface of the gel layer, the place where the gold nanoparticles generate heat by light irradiation is far from the cell, so that it is possible to reduce cell damage due to heat.
(3) In the cell culture container 1 of the embodiment, the cell culture container 1 has the plurality of wells 11, and the well 11 has a bottom. Accordingly, the cell culture substrate detached from the well 11 can be suspended in the fluid such as the culture solution or the buffer solution, so that it is possible to recover the cell culture substrate 14 having the cell 300 adhering thereto together with the fluid.
(4) In the cell culture container 1 of the embodiment, the cell culture container 1 has the plurality of wells 11, and the well 11 has the through-hole 12 at the bottom of the cell culture container 1. Accordingly, it is possible to recover the cell culture substrate 14 which has been contracted and has the selected cell 300 adhering thereto through the through-hole 12 from the bottom of the cell culture container by the suction pump or the like.
(5) In the cell culture container of the embodiment, the cell culture container 1 has the plurality of wells 11, and the well 11 has a diameter such that about one cell can be placed per well. Accordingly, the recovery of the desired cells is facilitated.
(6) In the cell culture container of the embodiment, the gel 15 is a gelatin gel or a collagen gel. Accordingly, manufacturing, handling, and the like are facilitated.
(7) The method for manufacturing a cell culture container in the embodiment includes a step of filling the cell culture container 1 with the gel 15 capable of being denatured by heating, and a step of forming the gold nanoparticle layer 13 on one surface of the gel. In a case where the cell culture container has the plurality of wells, the filling of each well with the cell culture substrate in which the gold nanoparticles are dispersed is typically performed by preparing a gel, in which the gold nanoparticles are dispersed, in a large container and pressing the cell culture container against the gel. It is difficult to fill all the wells with the prepared gel, and loss of the gel occurs by the amount of gel protruding from the well. Since the gold nanoparticles are dispersed in the gel, loss of the gold nanoparticles also occurs with the loss of the gel. However, by filling the cell culture container 1 with the gel and forming the gold nanoparticle layer on one surface of the gel, compared to the case of filling the cell culture container with the cell culture substrate in which the gold nanoparticles are dispersed, it is possible to obtain a cell culture container with which the selected cell 300 can be recovered without wasting the gold nanoparticles.
(8) In the method of manufacturing a cell culture container in the embodiment, the cell culture container 1 has the plurality of wells 11, each of the wells 11 is filled with the gel 15, and the gold nanoparticle layer 13 is formed on one surface of the gel. Accordingly, it is possible to obtain a cell culture container with which the selected cell 300 can be recovered without wasting the gold nanoparticles.
(9) The method for manufacturing a cell culture container in the embodiment includes a step of forming the gold nanoparticle layer 13 in the wells of the cell culture container 1 having the plurality of wells, and a step of forming the gel layer capable of being denatured by heating, on the gold nanoparticle layer 13. Accordingly, it is possible to obtain a cell culture container with which the selected cell 300 can be recovered without wasting the gold nanoparticles.
(10) The cell recovery system in the embodiment includes the cell culture container 1 and the suction pump 40 connected to the cell culture container 1. Accordingly, it is possible to suction the selected cell 300 or the cell culture substrate 14 having the selected cells 300 adhering thereto by the suction pump 40, and it is possible to recover easily the selected cell 300 or the cell culture substrate 14 having the selected cell 300 adhering thereto.
(11) The method for acquiring a cell in the embodiment includes a step of selecting the cell 300 to be acquired from the cells placed in the cell culture container 1, a step of irradiating the cell culture substrate 14 in the vicinity of the selected cell 300 with light, and a step of recovering the selected cell. Accordingly, it is possible to recover the selected cell 300 without damaging the selected cell 300.
(12) The method for acquiring a cell of the embodiment includes a step of selecting the cell 300 to be acquired from the cells respectively placed in the wells 11 of the cell culture container 1, a step of irradiating the cell culture substrate 14 filling the well 11 in which the selected cell 300 is placed with light, a step of taking out the cell culture substrate 14, and a step of recovering the cell from the cell culture substrate 14. Accordingly, the selected cell 300 can be recovered together with the cell culture substrate 14. Therefore, it is possible to increase the number of cells recovered per unit time without damaging the cells 300.
(13) In the method for acquiring a cell of the embodiment, the step of taking out the cell culture substrate 14 filling the well 11 is a step of suctioning the cell culture substrate 14 filling the well 11 with the suction pump or the like. Accordingly, it is possible to recover the cell culture substrate 14 having the selected cells 300 adhering thereto by suction, and it is possible to recover the selected cell 300 without damaging the selected cell 300.
(14) In the method for acquiring a cell in the embodiment, the step of taking out the cell culture substrate 14 is a step of pouring the fluid such as the culture solution or the buffer solution into the cell culture container 1. Accordingly, it is possible to recover the cell culture substrate 14 having the selected cell 300 adhering thereto together with the fluid, and it is possible to recover the selected cell 300 without damaging the selected cell 300.
(15) In the method for acquiring a cell of the embodiment, the step of recovering the selected cell 300 from the cell culture substrate 14 is a step of adding collagenase to the cell culture substrate 14. Accordingly, it is possible to recover the selected cell 300 from the cell culture substrate 14 without damaging the selected cell 300.

The present invention is not limited to the contents of the above embodiments. Other embodiments that are conceivable within the scope of the technical idea of the present invention are also included within the scope of the present invention.

### (Examples]

Cells of the present embodiment were obtained using HeLa cells as cells.

### (Reagent Preparation)

- PBS (-)
   NaCl (4.003 g), KCl (0.1003 g), KH₂PO₄ (0.1006 g), and NaH₂PO₄ (0.575 g) were dissolved in deionized water (500 mL) and then sterilized in an autoclave to prepare PBS(-).
- 10 times inorganic salt culture medium
   Anhydrous CaCl₂ (20.2 mg), MgCl·6H₂O (23.5 mg), KCl (40.4 mg), NaCl (639.7 mg), and NaH₂PO₄·2H₂O (14.1 mg) were added to and dissolved in deionized water (10 mL) to prepare a 10 times inorganic salt culture medium.
- Reconstituted solution
   NaHCO₃ (219.7 mg) and HEPES (477.12 mg) were added to and dissolved in NaOH (0.05 M, 10 mL) to prepare a reconstituted solution.
- Dilute hydrochloric acid
   A dilute hydrochloric acid (pH 3.0) was prepared by adjusted 0.05 M HCl aqueous solution to pH 3.0 using a pH meter (manufactured by HORIBA, Ltd., pH/COND METER D-54). Here, each of the 10 times inorganic salt culture medium, the reconstituted solution, and dilute hydrochloric acid (pH 3.0) was filter-sterilized using a filter (manufactured by ADVANTEC, pore size of 0.20 µm).
- Concentrated gold nanoparticles (AuNP) solution
   2 mL (1 mL×2) of Growth solution [refer to S. Yagi, et al, J Electrochem Soc, 159, H668 (2012)], which was a solution having grown gold nanoparticles were input into a centrifuge tube and centrifuged at 25°C and at 3000 rpm for 20 minutes. The supernatant solution (0.9 mL × 2) was removed, and ultrapure water (0.3 mL × 2) was added, and a concentrated AuNP solution (Au 750 µM) was prepared.

### (Preparing Collagen Solution)

On a clean bench (Showa Science Co., Ltd., S-1001PRV), collagen gel solution (2.1 mL) was prepared by adding the dilute hydrochloric acid (0.42 mL), the 10 times inorganic salt culture medium (0.20 mL), and the reconstituted solution (0.20 mL) to a cell culture substrate [manufactured by Nitta Gelatin Inc., Cellmatrix (registered trademark) I-A, collagen concentration of 0.3 wt%, pH 3.0, 0.56 mL] in this order under ice cooling.

### (Preparation of Well Type Microarray (Cell Culture Container))

An acrylic microarray in which wells having 1020 bottoms were formed was prepared. The diameter and depth of the wells were set to 200 µm, the distance between the centers of adjacent wells was set to 330 µm, and the distance from the bottom of each well to the bottom of the microarray was set to about 800 µm. The microarray was substituted with ethanol, and subsequently substituted with pure water. The concentrated gold nanoparticle (AuNP) solution prepared above was placed on the bottom of each well and left at 37°C overnight. Furthermore, each well was substituted with 200 µL collagen solution prepared above three times, and the well was filled with collagen gel. This was incubated in a direct heat CO₂ incubator at 37°C for 30 minutes.

### (Preparation of Through-Hole Type Microarray (Cell Culture Container))

An acrylic microarray in which wells having through-holes at 1020 bottoms were formed was prapared. The diameters of the wells and the through-holes were set to be equal to 200 µm, the depth of the wells was set to 200 µm, and the distance between the centers of adjacent wells was set to 330 µm. 1.8 mL of the embedded collagen solution prepared above was added to a 35-mm culture dish, and this was incubated in a direct heat CO₂ incubator at 37°C for 30 minutes to prepare a gel. Next, the bottom surface of the microarray was pressed against the above gel and each well was filled with collagen gel. Thereafter, the concentrated gold nanoparticle (AuNP) solution prepared above was placed above a collagen gel and left at 37°C overnight. Then, the microarray was inverted and the surface where the gel was exposed was used as a cell culture surface.

### (Cell Seeding and Detachment)

HeLa cells (6000 cells/microarray) dispersed in DMEM were poured into the prepared cell culture container, and cultured at 37°C for one day, and then the culture medium was removed by suction. Under a microscope, spot diameter of light irradiation was set so as to be minimum, and light irradiation was performed on a 37°C thermoplate on which an aluminum sheet was placed or in a cell culture chamber.

Light irradiation was performed using a device in which a laser beam source (manufactured by SIGMAKOKI Co., LTD., wavelength 532 nm, output 50 mW) was incorporated via a photoactivation (PA) epifluorescence device (manufactured by Nikon Corporation, Tl-PAU), and a mirror unit (manufactured by Nikon Corporation, TRITC) having a light transmittance of wavelength 532 nm was equipped, and fluorescence/phase contrast observation and imaging of cells were performed using an inverted fluorescence microscope (manufactured by Nikon Corporation, ECLIPSE Ti-U) and imaging sensor control software (manufactured by WRAYMER INC, WraySpect). The observation was performed using a 4-fold and 10-fold objective lens (manufactured by Nikon Corporation, Plan-Fluor).

### (Result)

In the well type microarray, the gel was denatured by light irradiation for 30 seconds and detached from the microarray. In a case where gold nanoparticles were uniformly dispersed and embedded in the gel, it took 60 seconds (data not shown) for denaturation of the gel. Therefore, the irradiation time was reduced to half by the cell culture container of the present invention.

In the through-hole type microarray, in a case where gold nanoparticles were uniformly dispersed and embedded in the gel, the gel was denatured by light irradiation for eight seconds, but HeLa cells, which are sensitive to heat, were killed even by irradiation for one second (data not shown). On the other hand, in the method of the present invention, the gel could be denatured by light irradiation for two seconds, and the cells were also alive.

### [Reference Signs List]

1 Cell culture container
7 Laser beam
10 Cell culture container body
11 Well
12 Through-hole
13 Gold nanoparticle layer
14 Cell culture substrate
15 Gel
20 Culture solution
30 Cell
40 Suction pump
70 Microscope
300 Selected cell
1000 Cell recovery system

## Claims

1. A cell culture container filled with a cell culture substrate including a gel layer formed of a gel capable of being denatured by heating and a gold nanoparticle layer formed on one surface of the gel layer,
wherein a cell is cultured on a side of the one surface or a side of the other surface in the cell culture substrate.

2. The cell culture container according to Claim 1,
wherein the cell culture container is a cell culture container having a plurality of wells, and
each of the wells is filled with the cell culture substrate.

3. The cell culture container according to Claim 2,
wherein the well has a bottom.

4. The cell culture container according to Claim 2,
wherein the well has a through-hole at the bottom.

5. The cell culture container according to Claim 4,
wherein the diameter of the through-hole is equal to or smaller than the diameter of the well.

6. The cell culture container according to any one of Claims 2 to 5,
wherein the well has a diameter such that about one cell is placed per well.

7. The cell culture container according to any one of Claims 2 to 6,
wherein the wells are arranged in an array.

8. The cell culture container according to any one of Claims 1 to 7,
wherein the gel is a collagen gel or a gelatin gel.

9. A method for manufacturing a cell culture container, comprising:
a step of filling a cell culture container with a gel capable of being denatured by heating; and
a step of forming a gold nanoparticle layer on one surface of the gel.

10. The method of manufacturing a cell culture container according to Claim 9,
wherein the cell culture container is a cell culture container having a plurality of wells, and
the step of filling the cell culture container with the gel is a step of filling each of the wells with the gel.

11. The method of manufacturing a cell culture container according to Claim 9 or 10,
wherein the step of forming the gold nanoparticle layer on one surface of the gel comprises
a step of placing a solution containing gold nanoparticles above the gel, and a step of evaporating a liquid component in the solution.

12. The method of manufacturing a cell culture container according to Claim 10,
wherein each of the plurality of wells has a through-hole at the bottom, and the step of forming the gold nanoparticle layer on one surface of the gel comprises
a step of placing a solution containing gold nanoparticles above the gel with a cell culture surface of the cell culture container facing downward,
a step of evaporating a liquid component in the solution, and
a step of turning the cell culture container upside down.

13. A method of manufacturing a cell culture container, comprising:
a step of forming a gold nanoparticle layer in wells of a cell culture container having a plurality of the wells; and
a step of forming a gel layer capable of being denatured by heating above the gold nanoparticle layer.

14. A cell recovery system, comprising:
the cell culture container according to any one of Claims 1 to 8; and
a suction pump.

15. A method of acquiring a cell, comprising:
a step of selecting a cell to be acquired from cells placed in the cell culture container according to any one of Claims 1 to 8;
a step of irradiating a cell culture substrate in the vicinity of the selected cell with light; and
a step of recovering the selected cell.

16. A method of acquiring a cell, comprising:
a step of selecting a cell to be acquired from cells respectively placed in the wells of the cell culture container according to any one of Claims 2 to 8;
a step of irradiating a cell culture substrate filling the well in which the selected cell is placed, with light;
a step of taking out the cell culture substrate irradiated with light from the cell culture container; and
a step of recovering the cell from the cell culture substrate taken out from the cell culture container.

17. The method of acquiring a cell according to Claim 16,
wherein the step of taking out the cell culture substrate from the cell culture container is a step of suctioning the cell culture substrate irradiated with light.

18. The method of acquiring a cell according to Claim 16,
wherein the step of taking out the cell culture substrate from the cell culture container is a step of suspending the gel irradiated with light in a liquid in the cell culture container and recovering the liquid.
